# EUROPEAN PATENT SPECIFICATION

(11) **EP 2 125 861 B1**
(45) Date of publication and mention of the grant of the patent: **16.07.2014**
(21) Application number: 07861342.9
(22) Date of filing: 19.09.2007
(51) Int. Cl.: C07K 14/775

(54) **METHOD OF PURIFYING APOLIPOPROTEIN A-1**
VERFAHREN ZUR AUFREINIGUNG VON APOLIPOPROTEIN A-1
PROCÉDÉ DE PURIFICATION DE L'APOLIPOPROTÉINE A-1

(30) Priority: 20.12.2006 CN 200610147503
(43) Date of publication of application: 02.12.2009
(73) Proprietor: Hoang, Kieu, Agoura Hills, CA 91301 (US)
(72) Inventor: XIANGFEI, Bao, 200245 Shanghai (CN); HOANG, Kieu, Agoura Hills, CA 91301 (US)
(74) Representative: Eisenführ Speiser
(86) International application number: PCT/US2007/020258
(87) International publication number: WO 2008/088403

(56) References cited:
- US-A- 5 525 472
- US-A- 5 834 596
- US-A1- 2006 014 692
- US-B1- 6 423 830
- MEZDOUR H ET AL: "ANION-EXCHANGE FASR PROTEIN LIQUID CHROMATOGRAPHIC CHARACTERIZATION AND PURIFICATION OF APOLIPOPROTEINS A-I, A-II, C-I, C-II, C-III0, C-III1, C-III2 AND E FROM HUMAN PLASMA", JOURNAL OF CHROMATOGRAPHY, ELSEVIER SCIENCE PUBLISHERS B.V, NL, vol. 414, no. 1, 20 February 1987 (1987-02-20), pages 35-45, XP009023395, ISSN: 0021-9673
- SANZ ET AL.: 'A New Vaccinia Virus Intermediate Transcription Factor' J. VIROL. vol. 72, no. 8, August 1998, pages 6880 - 6883, XP008108866
- 'Ion Exchange Chromatography & Chromatofoccusing: Principles and Methods' AMERSHAM BIOSCIENCES 2004, XP008133489
- PERSSON ET AL.: 'Purification of recombinant apolipoprotein A-1Milano expressed in Escherichia coli using aqueous two-phase extraction followed by temperature-induced phase separation' J. CHROMATOGRAPHY B vol. 711, 1998, pages 97 - 109, XP002930071

## Description

### FIELD OF THE INVENTION

The present invention relates to protein preparation, and especially the preparation of apoA-1.

### BACKGROUND OF THE INVENTION

High Density Lipoprotein (HDL) is an important lipoprotein in blood. It participates in a process called Reverse Cholesterol Transport (RCT), through which cholesterol in tissue cells can be transported to the liver to be metabolized into a harmless substance, hence restraining the occurrence and evolution of atherosclerosis (AS). Apolipoprotein A-1 (apoA-1) is the main form of apolipoprotein in High Density Lipoprotein (HDL). It is closely related to the physiological function of HDL in the blood. It is the main undertaker of the HDL anti-atherosclerosis function. Besides, according to recent research results, apoA-1 deficiency may cause the evolution of atherosclerosis and an increase of inflammation. Furthermore, apoA-1 decreases Low Density Lipoproteins (LDL) and cleans plaque. In addition, according to recent research results, apoA-1 is promising for applications in drugs with an anti-inflammation effect or liver-targeting function.

Methods such as ultra-speed centrifuge, organic solvent precipitation, and high performance liquid chromatography (HPLC) are usually used to purify apoA-1. However, there are some innate defects in these methods, such as low yield, high cost, insecurity, and a production scale that is too small. These methods are not suitable for apoA-1 industrial production.

On the other hand, as one of the fractions acquired after plasma fractionation, plasma fraction IV is always discarded because no useful product can be purified for commercial application. In accordance with the present invention, a purification method suitable for large-scale production is provided, and apoA-1 with high purity is acquired from plasma fraction IV.

Hafid Mezdour et al. (Journal of Chromatography, 414 (1987) 35-45) describe a procedure for the rapid isolation of urea-soluble apolipoproteins (apo) from delipidated human very-low- and high-density lipoproteins using anion-exchange fast protein liquid chromatography.

US 6,423,830 B1 discloses a process for purifying apolipoprotein A (ApoA) or apolipoprotein E (ApoE) from human plasma, by obtaining a fraction of human plasma containing said ApoA or ApoE, prepurifying said fraction in at least one step, binding said ApoA or ApoE to an anion exchange chromatography gel, and thereafter eluting said ApoA or ApoE from said anion-exchange chromatography gel. The thus produced ApoA or ApoE can be used for the manufacture of a medicament in the treatment of atherosclerosis and cardiovascular diseases, or peripheral atherosclerosis and sepsis as well as in a method for treatment of atherosclerosis and cardiovascular diseases, or peripheral atherosclerosis and sepsis when administered in a therapeutically effective amount.

### SUMMARY OF THE INVENTION

According to the present invention, it was found that urea can dramatically influence the behavior of apoA-1 in ion exchange chromatography. When apoA-1 is not combined with urea, it is very easily absorbed on an anion exchange column and relatively difficult to be eluted off the column. However, when apoA-1 is combined with urea, it is very easy to be eluted off an anion exchange column. Therefore, according to the present invention, apoA-1 is purified by two anion exchange columns, using two different elution profiles.

The present invention provides a method of purifying apolipoprotein A-1, including the following steps: a) plasma fraction IV (acquired by the Cohn Ethanol Fractionation method) is mixed with a 1-8 M urea solution, forming a fraction IV pretreatment solution; b) the pretreatment solution acquired in step a is loaded to a first anion exchange chromatography column, and then eluted by a 1-8 M urea solution to obtain apoA-1 protein; c) apoA-1 protein solution from step b is loaded in a second anion exchange chromatography column, and then eluted by 0-1 M urea solution to obtain pure apoA-1 protein. This method has advantages such as high yield, low cost, and suitability for industrial production. Besides, this method uses plasma fraction IV as its raw material, thereby making full use of plasma resources.

The pure apoA-1 acquired in this invention is promising for applications in atherosclerosis treatment, anti-inflammation treatment, antitoxin treatment, liver-targeting drugs, etc.

### DETAILED DESCRIPTION OF THE DRAWINGS

Fig. 1 is a flow chart showing the production process of apoA-1 from plasma fraction IV.
Fig. 2 shows the elution process of the first ion exchange chromatography in Example 1.
Fig. 3 shows the SDS-PAGE electrophoresis result of the sample taken in the first ion exchange chromatography in Example 1.
Fig. 4 shows the SDS-PAGE electrophoresis result of the sample taken in the second ion exchange chromatography in Example 1.
Fig. 5 shows SDS-PAGE electrophoresis result of the sample taken in Example 4.
Fig. 6 shows SDS-PAGE electrophoresis result of the sample taken in Example 2.
Fig. 7 shows SDS-PAGE electrophoresis result of the sample taken in Example 3.

### DETAILED DESCRIPTION OF THE PREFERRED EMBODIMENT

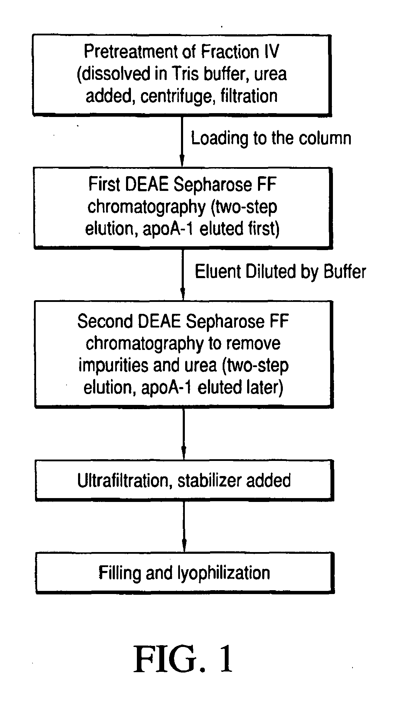

An embodiment of the process according to the present invention is illustrated in Fig. 1, which is a flow chart showing the production process of apoA-1 from plasma fraction IV.

### (1) plasma fraction IV pretreatment

The fraction IV described is acquired by the Cohn Ethanol Fractionation method (Cohn, E. J.; Strong, L.E.; Huges, W.L.; et al., Preparation and properties of serum and plasma proteins IV. A system for the separation into fractions of the protein and lipoprotein components of biological tissues and fluids. Amer Chem Soc., 1946, 68:459-475). The fraction IV is dissolved in a buffer, and a certain amount of urea is added to the solution and mixed thoroughly. In this process the apoA-1 is combined with urea, and this combination is reversible. The concentration of the urea added is 1-8 M, preferably 3-7 M, and more preferably 5-6 M. The mass ratio of the fraction IV and the urea is 1:30-300, preferably 1:90-240, and more preferably 1:150-210.

A buffer that is usually used in this field was chosen: Tris buffer, phosphate buffer or HEPES buffer, preferably Tris buffer. The buffer pH is 7.2-8.5, preferably 7.5-8, and more preferably 7.8.

In another example, the fraction IV is dissolved under low temperature (0-4°C).

In another example, the pretreatment solution is centrifuged to remove sediments, and then filtered. The centrifuge speed is 6,000-10,000 rpm, preferably 8,000 rpm, and the pore size of the filtration membrane is 0.2 - 0.6 µ m, preferably 0.45 µ m.

### (2) The first DEAE anion exchange chromatography

The solution obtained in step (1) is loaded to a DEAE (Diethylamino Ethanol) anion exchange column, and protein in the solution, including apoA-1, is combined onto the column. More specifically, the apoA-1 solution acquired in step (1) is diluted with water 1-10 fold, and preferably 3-7 fold. After dilution, the solution is loaded onto the anion exchange column at a flow rate of 0.5-1.5 ml/min, preferably 0.8-1.2 ml/min.

Then, the protein is eluted by two elution steps. In the first step, a buffer with low conductivity is used to elute the column. ApoA-1 combines with the column weakly at this time, and protein mainly containing apoA-1 can be eluted first. In the second step, a buffer with high conductivity is used to elute the column, and protein mainly containing impurities is eluted.

The eluent that elutes apoA-1 contains 1-8 M urea, preferably 3-7 M, and more preferably 5-6 M. The conductivity is 1-4 mS/cm, preferably 2-3.8 mS/cm, and more preferably 2.5-3.6 mS/cm. Salts in the eluent may include NaCl, KCl MgCl₂ and CaCl₂. NaCl is preferred.

The eluent that elutes impurities contains 0-1 M urea; 0 M is preferred. The conductivity is 4.5 - 100 mS/cm. Salts in the eluent may include NaCl, KCl, MgCl₂ and CaCl₂, NaCl is preferred.

A buffer usually used in this field is chosen: Tris buffer, phosphate buffer or HEPES buffer, preferably Tris buffer. The buffer pH is 7.2-8.5, preferably 7.5-8, and more preferably 7.8

The column flow rate of the fraction pretreatment solution obtained in step 1 is 0.5-1.5 ml/min, preferably 0.8-1.2 ml/min. The volume ratio of fraction IV and the column is 1:5-50, preferably 1:15-40, and more preferably 1:20-30:

### (3) The second DEAE anion exchange chromatography

The apoA-1 solution acquired in the first DEAE anion exchange chromatography step, containing apoA-1, urea and a slight amount of impurities, is diluted by water to a lower conductivity and then loaded onto a second DEAE column.

The protein is combined on the second DEAE column, and the urea remains in the solution and is eliminated as flow-through. Then, the protein is eluted by two elution steps. In the first step, a buffer with relatively low conductivity is used to elute the column. ApoA-1 strongly combines with the column, and the impurities are eluted off the column. In the second step, a buffer with higher conductivity is used to elute the column, and pure ApoA-1 is eluted.

The conductivity of the eluent that elutes the impurities is 1-20 mS/cm, preferably 7-15 mS/cm, more preferably 9-12 mS/cm, 0-1 M urea may exist in the eluent, and 0 M is preferred. Salts in the eluent may include NaCl, KCl, MgCl₂ and CaCl₂. NaCl is preferred.

The conductivity of the eluent that elutes the apoA-1 is 50-100 mS/cm, preferably 70-95 mS/cm, and more preferably 80-90 mS/cm. 0-1 M urea may exist in the eluent, and 0 M is preferred. Salts in the eluent may include NaCl, KCl, MgCl₂ and CaCl₂. NaCl is preferred.

In another example, after apoA-1 and a slight amount of impurities are combined on the second DEAE column, a conductivity gradient of 3 mS/cm/min is applied to elute the protein. The conductivity of the eluent rises from 1 to 100 mS/cm. Pure apoA-1 is eluted at a conductivity between 30 and 60 mS/cm and collected.

A buffer usually used in this field is chosen: Tris buffer, phosphate buffer or HEPES buffer, preferably Tris buffer. The buffer pH is 7.2-8.5, preferably 7.5-8, more preferably 7.8.

In this invention, post processing methods are also provided to process the pure apoA-1 solution acquired in step (3). The methods include an ultra-filtration step, a stabilizer adding step, and a lyophilization step. Freeze dried apoA-1 is ultimately acquired after post processing steps.

Ultra-filtration, which is frequently applied in this field, is used for apoA-1 solution conditioning, adjusting the solution to an appropriate pH and protein concentration. For the ultra-filtration, a Millipore PES ultra-filtration membrane with a molecular weight cutoff of 5000 is used, the working temperature is 4°C, and the working pressure is under 0.3 Mpa.

The stabilizer added is from among those frequently used in this field, including hydroxypropyl cellulose, hydroxypropyl methylcellulose, sodium cellulose glycolate, sucrose, sorbierite, etc.

A freeze drying method that is frequently applied in this field is used: the product is frozen below -36°C for 3-4 hours, and then freeze dried under a vacuum of 7-9 Pa.

The temperature in the cold trap is about -55°C, and then, in a second period, the shelf temperature is 40°C and the time is about 15 hours.

The chromatography columns used in this invention may be from among those frequently applied in this field, and the chromatography media may be QAE (quaternary amine) or DEAE anion exchange chromatography media, preferably DEAE.

The invention is further illustrated in detail in the following examples. For those experiments in the following examples, if a condition is not specified, it means that the condition is routine or advised by the manufacturer. Unless otherwise specified, the ratios mentioned below are mass ratios.

Unless defined otherwise, the definitions of the technical or scientific terms are those generally understood by the technician of ordinary skill in this field.

### EXAMPLE 1 ApoA-1 Purification

The starting materials include: 0.2 g plasma fraction IV, two 5 ml DEAE anion exchange columns (from GE Healthcare), and purification and conductivity determination equipment, namely, an AKTA EXPLORER 100 (from GE Healthcare).

### (1) plasma fraction IV pretreatment

0.2 g of fraction IV is dissolved in 100 ml Tris buffer (pH 7.8, 4°C). Urea is added to the solution until the end concentration reaches 6 mol/L and is mixed thoroughly. The solution is centrifuged at 8,000 rpm to remove sediment, then filtrated by a 0.45 µm filter.

### (2) The first DEAE anion exchange chromatography

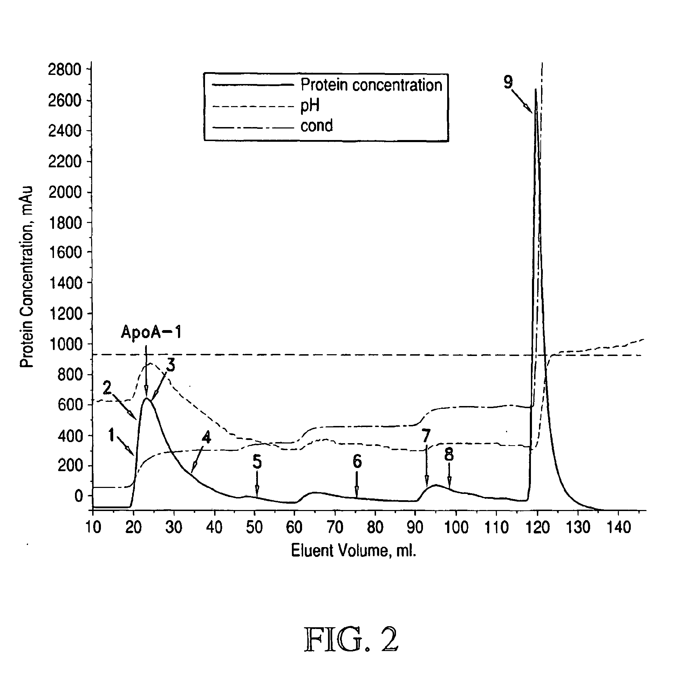

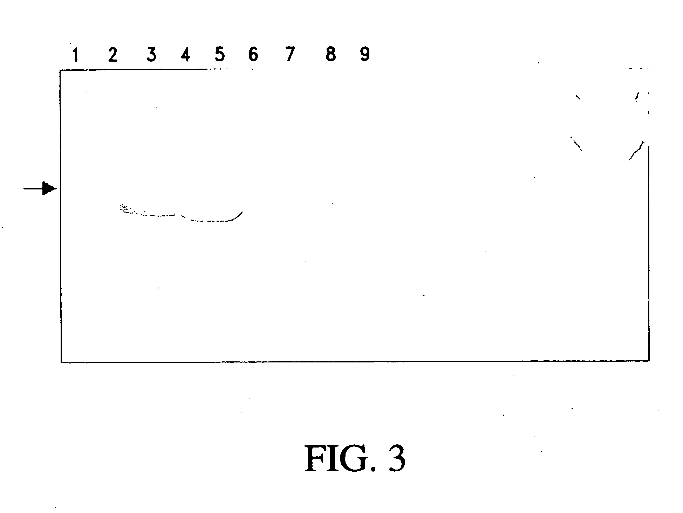

A first DEAE column is equilibrated by Tris buffer (pH 7.8) containing 6 mol/L urea. The solution from step (1) is loaded onto the DEAE column at a flow rate of 1 ml/min, Tris buffer (pH 7.8, conductivity 3.5 mS/cm) with a urea concentration of 6 mol/L is used to elute the column. ApoA-1 is eluted. The eluted volume is 20 ml. Four different Tris buffers (each with pH 7.8, and with conductivities of 4.3 mS/cm, 5.4 mSs/cm, 6.7 mSs/cm, and 59.2 mSs/cm, respectively) are used to elute the column, and the impurities are eluted. The elution process and SDS-PAGE electrophoresis results are shown in Figs. 2 and 3, respectively. The x-coordinate in Fig. 2 is the elute volume in the chromatography process, and the y-coordinate is the protein concentration in the elute. The numbers 1 to 9 indicate the spots where the sample is taken. The sample is determined by SDS-PAGE electrophoresis in Fig. 3. The arrow shows the position of the apoA-1 protein.

### (3) The second DEAE anion exchange chromatography

The apoA-1 eluted in step (2) (pH 7.8, conductivity 3.5 mS/cm), containing ApoA-1, urea and slight amount of impurities, is diluted with water by 5 fold and loaded into the second DEAE column at a flow rate of 10 ml/min. ApoA-1 and a slight amount of impurities are combined on the column, and urea remains in the solution and is removed as flow-through.

Tris buffer without urea (pH 7.8, conductivity 11.7 mS/cm) is used to elute the column, and the impurities are eluted off the column. Then, Tris buffer without urea (pH 7.8, conductivity 85.2 mS/cm) is used to elute the column, and apoA-1 is eluted off.

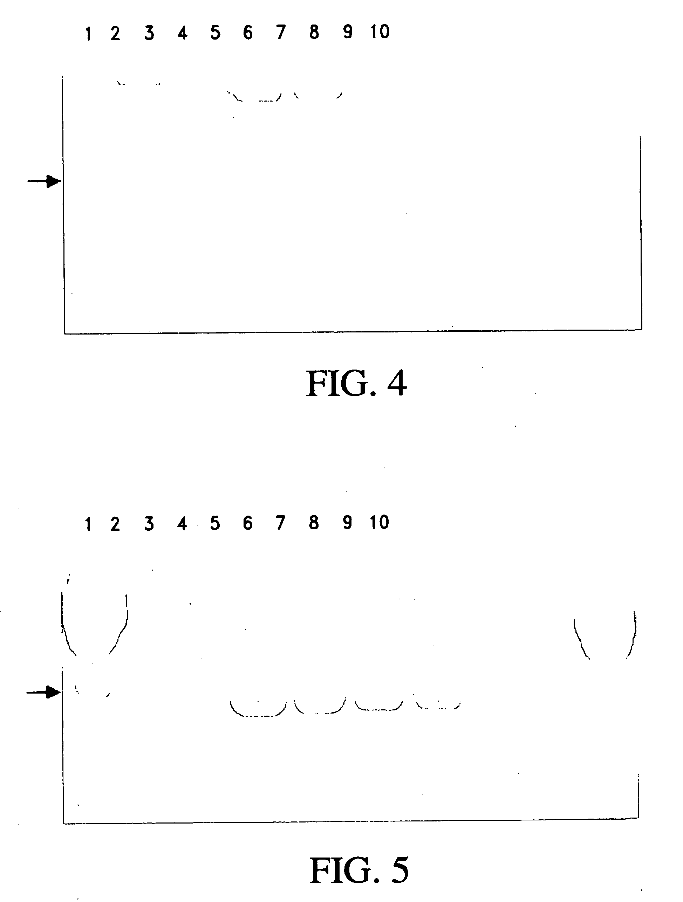

The SDS-PAGE electrophoresis results show that pure apoA-1 is acquired in this step (Fig. 4). The sample 1 is the protein in the column flow-though solution during the loading process. The sample 2 is the protein mark. The numbers 3 to 5 indicate the sample collected during the first elution process, which mainly consists of impurities. The numbers 6 to 10 indicate the sample collected during the second elution process, which mainly consists of apoA-1. Samples 6 and 7 are diluted by 5 fold, and sample 8 is diluted by 10 fold. The arrow shows the position of apoA-1 protein.

### EXAMPLE 2 ApoA-1 Purification

The 0.2 g of plasma fraction IV and the equipment are the same as those in

### Example 1.

### (1) plasma fraction IV pretreatment

The 0.2 g of fraction IV is dissolved in 100ml Tris buffer (pH 7.8, 4°C). Urea is added to the solution until the end concentration reaches 6 mol/L, and the solution is mixed thoroughly. The solution is centrifuged at 8,000 rpm to remove the sediment and then filtrated by a 0.45 µm filter.

### (2) The first DEAE anion exchange chromatography

The first DEAE column is equilibrated by Tris buffer (pH7.8) containing 1 mol/L urea. The solution from step (1) is loaded onto the first DEAE column at a flow rate of 1 ml/min. Tris buffer (pH 7.8, conductivity 3.5 mS/cm) with a urea concentration of 1 mol/L is used to elute the column. ApoA-1 is eluted. The elute volume is 20 ml. Tris buffer (pH 7.8, 59.2 mS/cm respectively) is used to elute the column, and the impurities are eluted.

### (3) The second DEAE anion exchange chromatography

The procedures are the same as in Example 1. The purification results are shown in Fig. 5. Sample 1 is the supernatant of plasma fraction IV after the pretreatment process. Samples 4 to 8 are apoA-1 collected in the purification process. Sample 10 is the impurities collected during the purification process. The arrow shows the position of ApoA-1 protein.

### EXAMPLE 3 ApoA-1 Purification

The 0.2 g of plasma-fraction IV and the equipment are the same as those in Example 1.

### (1) plasma fraction IV pretreatment

The 0.2 g of fraction IV is dissolved in 100 ml Tris buffer (pH 7.8, 4°C). Urea is added to the solution until the end concentration reaches 6 mol/L, and the solution is mixed thoroughly. The solution is centrifuge at 8,000 rpm to remove the sediment and then filtrated by the 0.45µm filter.

### (2) The first DEAE anion exchange chromatography

The first DEAE column is equilibrated by Tris buffer (pH 7.8) containing 8 mol/L urea. The solution from step (1) is loaded onto the DEAE column at a flow rate of 1 ml/min. Tris buffer (pH 7.8, conductivity 3.5 mS/cm) with 8 mol/L urea is used to elute the column. ApoA-1 is eluted. The elute volume is 20 ml. Tris buffer (pH 7.8, conductivity 59.2 mS/cm) is used to elute the column, and the impurities are eluted.

### (3) The second DEAE anion exchange chromatography

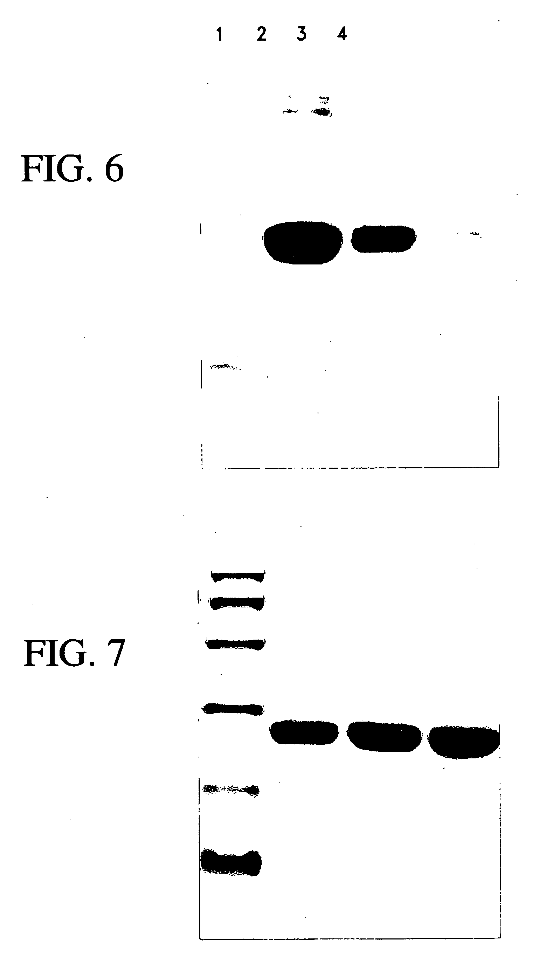

The procedures are the same as in Example 1. The purification results are shown in Fig. 7.

### EXAMPLE 4 ApoA-1 Pilot Scale Purification

The starting materials include: 60g of plasma fraction IV, two 1500 ml ion exchange columns (from Shanghai Jinhua Chromatography Equipment Cooperaration), anion exchange media, namely, DEAE Sepharose FF (from GE Healthcare), a pump and a UV detector (from Shanghai Jinhua Chromatography Equipment Corporation), and conductivity determination equipment, namely, an AKTA EXPLORER 100 (from GE Healthcare)

### (1) plasma fraction IV pretreatment

60g fraction IV is dissolved in 100ml Tris buffer (pH 7.8, 4°C). Urea is added to the solution until the end concentration reaches 6 mol/L, and the solution is mixed thoroughly, centrifuged at 8,000 rpm to remove the sediment, and then filtrated by the 0.45 µm filter.

### (2) The first DEAE anion exchange chromatography

A first DEAE column is equilibrated by Tris buffer (pH 7.8) containing 6 mol/L urea. The solution from step (1) is loaded onto the DEAE column at a flow rate of 10 ml/min. Tris buffer (pH 7.8, conductivity 3.7 mS/cm) with a urea concentration of 6 mol/L is used to elute the column. ApoA-1 is eluted. The eluted volume is 10L. Tris buffer (pH 7.8, conductivity 80.3 mS/cm) is used to elute the column, and the impurities are eluted.

### (3) The second DEAE anion exchange chromatography

The ApoA-1 eluted in step 2 (pH 7.8, conductivity 3.7 mS/cm), containing apoA-1, urea and slight amount of impurities, is diluted with water by 5 fold and loaded onto the second DEAE column at a flow rate of 10 ml/min. The apoA-1 and a slight amount of impurities are combined to the column, and urea remains in the solution and is eliminated as flow-through.

Tris buffer without urea (pH 7.8, conductivity 12.2 mS/cm) is used to elute the column, and the impurities are eluted off the column. Then, This buffer without urea (pH 7.8, conductivity 50.4 mS/cm) is used to elute the column, and apoA-1 is eluted off.

The SDS-PAGE electrophoresis results are shown in Fig. 5. The results show that pure apoA-1 can also be acquired in a pilot scale process.

### EXAMPLE 5 ApoA-1 Protein Determination

The apoA-1 protein purified in Example 1 is determined by an apoA-1 immunoturbity determination kit from Shanghai SUN Biotech Co. LTD.

10 µ 1 of purified protein is added to 1 ml of apoA-1 antiserum, and then incubated at 37°C for 15 minutes. After incubation, the solution is detected by 505 nm absorbency. The results show that this protein is ApoA-1.

### EXAMPLE 6 ApoA-1 Protein Determination

Using the method described in Example 5, the ApoA-1 proteins from Examples 2 and 3, respectively, and the apoA-1 protein from Example 4 are detected by the apoA-1 immunoturbity determination kit from Shanghai SUN Biotech Co. LTD.

Similar results are obtained.

## Claims

1. A method of purifying apolipoprotein A-1, comprising:
a) mixing plasma fraction IV with a solution of 1-8 M urea, forming a fraction IV pretreatment solution;
b) loading the pretreatment solution acquired in step a) to a first anion exchange chromatography column, and then eluting with a buffer I comprising 1-8 M urea having a conductivity of 1-4 mS/cm to obtain an eluent I that mainly contains apoA-1 protein;
c) loading eluent I to a second anion exchange chromatography column, first eluting with a buffer II, having a conductivity of 1-15 mS/cm, and later eluting with a buffer III, having a conductivity of 30-100 mS/cm, to obtain pure apoA-1 protein.

2. The method of claim 1, wherein buffer II includes 0-1 M urea.

3. The method of claim 1, wherein buffer III includes 0-1 M urea.

4. The method of claim 1, wherein the urea concentration in steps a) and b) is 3-8 M.

5. The method of claim 1, wherein the conductivity of buffer I is 2-4 mS/cm. the conductivity of buffer II is 7-15 mS/cm, and the conductivity of buffer III is 70-95 mS/cm.

6. The method of claim 1, wherein each anion exchange chromatography column is one of 1) a strong anion ion exchange chromatography column and 2) a weak anion ion exchange chromatography column.

7. The method of claim 1, wherein the mass ratio of fraction IV in step 1 and urea is 1:30-300.

8. The method of claim 1, wherein the buffers I, II, III of steps b) and c) contain at least one salt selected from the group consisting of NaCl, KCl, MgCl₂ and CaCl₂.

9. The method of claim 1, wherein, between steps a) and b), the fraction IV pretreatment solution is centrifuged to remove sediments, and then filtrated.

10. The method of claim 1, wherein, between steps b) and c), most impurities are eluted by a buffer IV having a conductivity of 4.5-100 mS/cm.

11. The method of claim 10, wherein buffer IV contains 0-1 M urea.

12. The method of claim 10, wherein buffer IV contains at least one salt selected from the group consisting of NaCl, KCl, MgCl₂ and CaCl₂.

13. The method of claim 1, wherein the pH of buffers I, II and III is 7.2-8.5.

14. The method of claim 1, wherein the pH of buffers I, II and III is 7.5-8.

15. The method of claim 1, further comprising, after step c), at least one of ultra filtering the pure apoA-1 protein, adding a stabilizer to the pure apoA-1 protein, and lyophilizing the pure apoA-1 protein.

## Patentansprüche

1. Verfahren zum Aufreinigen von Apolipoprotein A-1, umfassend:
a) Mischen der Plasmafraktion IV mit einer Lösung von 1-8 M Harnstoff unter Bildung einer Fraktion-IV-Vorbehandlungslösung;
b) Laden der in Schritt a) erhaltenen Vorbehandlungslösung auf eine erste Anionenaustausch-Chromatographiesäule und danach Eluieren mit einem 1-8 M Harnstoff enthaltenden Puffer I mit einer Leitfähigkeit von 1-4 mS/cm, so dass man einen Eluenten I erhält, der hauptsächlich apoA-1-Protein enthält;
c) Laden des Eluent I auf eine zweite Anionenaustausch-Chromatographiesäule, zuerst Eluieren mit einem Puffer II mit einer Leitfähigkeit von 1-15 mS/cm und später mit einem Puffer III mit einer Leitfähigkeit von 30-100 mS/cm, so dass man reines apoA-1-Protein erhält.

2. Verfahren nach Anspruch 1, wobei Puffer II 0-1 M Harnstoff enthält.

3. Verfahren nach Anspruch 1, wobei Puffer III 0-1 M Harnstoff enthält.

4. Verfahren nach Anspruch 1, wobei die Harnstoffkonzentration in den Schritten a) und b) 3-8 M beträgt.

5. Verfahren nach Anspruch 1, wobei die Leitfähigkeit von Puffer I 2-4 mS/cm, die Leitfähigkeit von Puffer II 7-15 mS/cm und die Leitfähigkeit von Puffer III 70-95 mS/cm beträgt.

6. Verfahren nach Anspruch 1, wobei jede Anionenaustausch-Chromatographiesäule jeweils eine ist aus 1) einer Starken-Anionenaustausch-Chromatographiesäule und 2) einer Schwachen-Anionenaustausch-Chromatographiesäule.

7. Verfahren nach Anspruch 1, wobei das Massenverhältnis von Fraktion IV in Schritt 1 und Harnstoff 1:30-300 beträgt.

8. Verfahren nach Anspruch 1, wobei die Puffer I, II, III der Schritte b) und c) mindestens ein Salz ausgewählt aus der Gruppe bestehend aus NaCl, KCl, MgCl₂ und CaCl₂ enthalten.

9. Verfahren nach Anspruch 1, wobei zwischen den Schritten a) und b) die FraktionIV-Vorbehandlungslösung zur Abtrennung von Sedimenten zentrifugiert wird und danach filtriert.

10. Verfahren nach Anspruch 1, wobei zwischen den Schritten b) und c) die meisten Verunreinigungen mit einem Puffer IV mit einer Leitfähigkeit von 4,5-100 mS/cm eluiert werden.

11. Verfahren nach Anspruch 10, wobei Puffer IV 0-1 M Harnstoff enthält.

12. Verfahren nach Anspruch 10, wobei Puffer IV mindestens ein Salz ausgewählt aus der Gruppe bestehend aus NaCl, KCl, MgCl₂ und CaCl₂ enthält.

13. Verfahren nach Anspruch 1, wobei der pH-Wert der Puffer I, II und III 7,2-8,5 ist.

14. Verfahren nach Anspruch 1, wobei der pH-Wert der Puffer I, II and III 7,5-8 ist.

15. Verfahren nach Anspruch 1, ferner umfassend nach Schritt c) wenigstens einen der Schritte Ultrafiltration des reinen apoA-1-Proteins, Zugeben eines Stabilisators zum reinen apoA-1-Protein und Lyophilisieren des reinen apoA-1-Proteins.

## Revendications

1. Procédé de purification de l'apolipoprotéine A-1, comprenant :
a) le mélange de la fraction IV d'un plasma avec une solution d'urée 1 à 8 M, de façon à former une solution de prétraitement de la fraction IV ;
b)le chargement de la solution de prétraitement obtenue à l'étape a) sur une première colonne chromatographique échangeuse d'anions, puis l'élution avec un tampon I comprenant de l'urée 1 à 8 M ayant une conductivité de 1 à 4 mS/cm pour obtenir un éluant I qui contient principalement la protéine apoA-1 ;
c)le chargement de l'éluant I sur une deuxième colonne chromatographique échangeuse d'anions, d'abord l'élution avec un tampon II ayant une conductivité de 1 à 15 mS/cm et ensuite l'élution avec un tampon III ayant une conductivité de 30 à 100 mS/cm, pour obtenir la protéine apoA-1 pure.

2. Procédé selon la revendication 1, dans lequel le tampon II comprend de l'urée 0 à 1 M.

3. Procédé selon la revendication 1, dans lequel le tampon III comprend de l'urée 0 à 1 M.

4. Procédé selon la revendication 1, dans lequel la concentration en urée dans les étapes a) et b) est de 3 à 8 M.

5. Procédé selon la revendication 1, dans lequel la conductivité du tampon I est de 2 à 4 mS/cm, la conductivité du tampon II est de 7 à 15 mS/cm et la conductivité du tampon III est de 70 à 95 mS/cm.

6. Procédé selon la revendication 1, dans lequel chaque colonne chromatographique échangeuse d'anions est une colonne parmi 1) une colonne chromatographique échangeuse d'anions forte et 2) une colonne chromatographique échangeuse d'anions faible.

7. Procédé selon la revendication 1, dans lequel le rapport massique de la fraction IV dans l'étape 1 à l'urée est de 1:30-300.

8. Procédé selon la revendication 1, dans lequel les tampons I, II et III des étapes b) et c) contiennent au moins un sel choisi dans le groupe constitué de NaCl, KCl, MgCl₂ et CaCl₂.

9. Procédé selon la revendication 1, dans lequel, entre les étapes a) et b), la solution de prétraitement de la fraction IV est centrifugée pour éliminer les sédiments avant d'être filtrée.

10. Procédé selon la revendication 1, dans lequel, entre les étapes b) et c), la plupart des impuretés sont éluées par un tampon IV ayant une conductivité de 4,5 à 100 mS/cm.

11. Procédé selon la revendication 10, dans lequel le tampon IV contient de l'urée 0 à 1 M.

12. Procédé selon la revendication 10, dans lequel le tampon IV contient au moins un sel choisi dans le groupe constitué de NaCl, KCl, MgCl₂ et CaCl₂.

13. Procédé selon la revendication 1, dans lequel le pH des tampons I, II et III est de 7,2 à 8,5.

14. Procédé selon la revendication 1, dans lequel le pH des tampons I, II et III est de 7,5 à 8.

15. Procédé selon la revendication 1, comprenant en outre, après l'étape c), au moins une étape parmi l'ultra-filtration de la protéine apoA-1 pure, l'addition d'un stabilisant à la protéine apoA-1 pure et la lyophilisation de la protéine apoA-1 pure.
